# EUROPEAN PATENT SPECIFICATION

(11) **EP 4 162 457 B1**
(45) Date of publication and mention of the grant of the patent: **29.01.2025**
(21) Application number: 21731322.0
(22) Date of filing: 07.06.2021
(51) Int. Cl.: G08B 21/14, G08B 21/02, G01N 27/16

(54) **PERSONAL SENSING DEVICE TO BE WORN BY A PERSON ON A SITE, AS WELL AS USE OF SUCH A PERSONAL SENSING DEVICE AND A SYSTEM COMPRISING ONE OR MORE OF SUCH PERSONAL SENSING DEVICES**
PERSÖNLICHE SENSORVORRICHTUNG ZUM TRAGEN DURCH EINE PERSON AN EINEM ORT SOWIE VERWENDUNG EINER SOLCHEN PERSÖNLICHEN SENSORVORRICHTUNG UND SYSTEM MIT EINER ODER MEHREREN SOLCHEN PERSÖNLICHEN SENSORVORRICHTUNGEN
DISPOSITIF DE DÉTECTION PERSONNEL DESTINÉ À ÊTRE PORTÉ PAR UNE PERSONNE SUR UN SITE, AINSI QUE L'UTILISATION D'UN TEL DISPOSITIF DE DÉTECTION PERSONNEL ET SYSTÈME COMPRENANT UN OU PLUSIEURS DESDITS DISPOSITIFS DE DÉTECTION PERSONNELS

(30) Priority: 09.06.2020 NL 2025789
(43) Date of publication of application: 12.04.2023
(73) Proprietor: Ansense Technologies B.V., 1521 RM Wormerveer (NL)
(72) Inventor: WELP, Jacobus Alfonsus Maria, 1521 RM WORMERVEER (NL)
(74) Representative: Algemeen Octrooi- en Merkenbureau B.V.
(86) International application number: PCT/NL2021/050361
(87) International publication number: WO 2021/251818

(56) References cited:
- WO-A1-2018/048517

## Description

### FIELD OF THE INVENTION

The present invention relates to a personal sensing device to be worn by a person on a site, comprising:
- a gas sensor for measuring a gas concentration of a gas;
- a communication system for communicating an alarm/event or operational condition, such as the gas concentration, to a computer system,
wherein the computer system is arranged for processing the communicated alarm/event and/or triggering an alarm/response in response to the communication of the operational condition, for instance when the computer system determines that the gas concentration exceeds a pre-determined limit.

The present invention further relates to the use of such a personal sensing device and a system comprising one or more of such personal sensing devices.

### BACKGROUND OF THE INVENTION

Such personal safety or sensing devices are known in the art. During operation of the personal sensing device, a sensor mechanism provides sensor data based on measurements of environmental conditions in an external environment that includes the personal sensing device. A control mechanism assesses if the environmental conditions indicate at least one of a set of threats, usually dangerously high gas concentrations. If yes, the control mechanism provides a corresponding alert. However, prior art personal sensing devices leave a lot to be desired in terms of safety, speed of communication and responsiveness to threats, in particular in case of unsafe working conditions, such as when hazardous materials are present, or in "lone worker" situations.

WO 2018/048517 A1 shows the pre-characterizing portion of claim 1. In particular the document discloses a gas sensing element. The gas sensing element may be used in a gas monitor or gas sensing device. The gas monitor or gas sensing device may be part of a lone worker safety system, preferably in an interconnected network of gas sensing devices. However, WO 2018/048517 A1 primarily employs "mesh" Wi-Fi to realize connectivity between the gas sensing devices and an alarm server. This may cause a lone worker to not have optimal network access and alarm server access at all times, possibly leading to dangerous situations.

An object of the present invention is therefore to make better use of the available resources and information, to make the site safer for persons at the site, in particular in "lone worker" situations or unsafe working conditions, such as when hazardous materials are present, to increase speed of communication and to improve responsiveness to threats.

### SUMMARY OF THE INVENTION

This object is achieved by a personal sensing device according to claim 1, the use of a personal sensing device according to claim 14 and a system of one or more personal sensing devices according to claim 15.

For instance, when the operational condition is compared to a desired or pre-determined status of the operational condition and the operational condition is found to be undesirable or otherwise unsatisfactory, such as not being within a desired range or exceeding a predetermined limit), in the sense that a fast and proportional action is determined for dealing with the respective operational condition leading to the alarm/event, e.g. to mitigate a threat. Or, for instance, when the alarm/event or operational condition communicated to the computer system indicates a "man-down" situation, the computer system will intelligently distribute instructions to nearby emergency services or nearby (safety) personnel to deal with the situation, for example by alerting on-site personnel to investigate the position of the person wearing the personal sensing device. A tailor-made, autonomous response is thus generated by the computer system, commensurate with the gravity of the operational condition/situation/alarm/event. In the context of this patent application, an alarm is the most serious form of an event, requiring immediate attention. In case of low battery, for example, the intelligent alarm/event instructions may comprise sending a message to the personal sensing device informing the person to recharge or replace the battery, i.e. alarm/event instructions are sent sufficient (and proportional) for resolving the situation. The above personal sensing device is particularly suitable for distribution of alarm/event instructions including emergency and evacuation alerts to nearby persons or group members.

Preferably, operational conditions, situations, alarms and/or events are saved or recorded in an events report, on an appropriate, preferably non-transitory, data storage medium, e.g. on the computer system. The events report may give an overview of e.g. alarm or event types occurring in the personal sensing device fleet, i.e. (all) the personal sensing devices at the site and/or other, related sites. The events report can be filtered by a person reading or studying the events report to see operational conditions, situations, alarms and/or events by a person wearing the personal sensing device, group, organization, alarm/event type or gas sensor type. The events report may show e.g. alarm/event trends over time, so that e.g. managers can track improvements in processes dealing with such operational conditions, situations, alarms and/or events. Such trends may be derived or analyzed by means of appropriate software applications, e.g. trend analysis software/programs.

Preferably, an events map report, an alerts report, an incident report, a usage and compliance report and/or a bump test and calibration report are also provided, on an appropriate, preferably non-transitory, data storage medium, e.g. on the computer system.

The events map may directly complement the events report, and shows where events have occurred on a map. Hovering over a point on the map will indicate the event type and its exact location on the site. The events map report can be used to observe clusters of points where events of a certain type may be occurring more frequently.

The alerts report may deliver a full breakdown of alerts or alarms occurring on the personal sensing devices, e.g. to see which personal sensing device user/wearer is triggering the most alerts or alarms, when and where alerts or alarms are occurring and what types of alerts or alarms are most common. Additionally, the time it takes for alerts or alarms to be resolved, resolution trends and the most common resolution reasons may be recorded in the alerts report. Alerts or alarms data may be saved in tabular format for easy export.

The incident report may be used to examine a particular incident that occurred at a specific point in time. Persons reading the incident report can see events mapped side-by-side over time for easy comparison and can also see where each of these events occurred on a map. These maps will help to determine the chronological order of events that occurred during an incident, and whether they may have been related. Such events may be analyzed and/or correlated by (appropriate) incident analysis software/programs, for instance installed on the computer system.

The usage and compliance report may show total personal sensing device usage per user (i.e. person wearing the personal sensing device), group or organization. The usage and compliance report may also show what percentage of this usage was done while the personal sensing device was compliant and/or as trending usage and compliance over time. The usage and compliance report may be used to review how team members (i.e. a group of persons wearing the personal sensing devices) use their personal sensing devices and e.g. to track changes in their usage over time. Usage and compliance may again be analyzed by (appropriate) usage and compliance software/programs, for instance installed on the computer system.

The bump test and calibration report may show how many bump tests and calibrations are performed on the device fleet, i.e. (all) the personal sensing devices at the site and/or other, related sites and show how many of these tests were successful and whether the tests were performed manually or with a dock. Additionally, the bump test and calibration report shows an overview of each gas sensor and hardware test, so that one can easily pinpoint which parts of the personal sensing device are working correctly (or not). The bump test and calibration report may again be analyzed by (appropriate) bump test and calibration software/programs, for instance installed on the computer system.

Furthermore, asset utilization reports may be generated, e.g. a beacons report, a devices and cartridges report and/or a docks report. The beacons report may communicate the status and effectiveness of "location beacons" (e.g. the personal sensing devices transmitting their respective locations).

The beacons report may monitor battery levels and ensure locations are being delivered frequently and consistently.

The devices and cartridges report may give a full overview of the status of the personal sensing devices, including their firmware version and when the personal sensing devices last communicated via the communication system. The devices an cartridges report may be used to e.g. stay on top of firmware updates and ensure the personal sensing devices and/or device cartridges are being used and maintained regularly.

Docks may automatically charge, bump test and calibrate the personal sensing devices. The docks report may deliver information on the locations and usage of the docks. A map may be provided to see where a dock was last used, and each dock's bump test may be tracked and calibration results may be used to track performance and ensure regular maintenance.

Again, the beacons report, devices and cartridges report, and/or docks report may be analyzed (e.g. for trends) by (appropriate) beacons report, devices and cartridges report, and/or docks report analysis software/programs, for instance installed on the computer system.

The operational condition may also concern the detection of hazardous materials with a suitable hazardous materials (LEL) sensor.

In an embodiment, the computer system is comprised by the personal sensing device or a mobile phone carried by the person in communication with the personal sensing device. Thus, the one or more alarm instructions can be determined and sent out by the personal sensing device itself or a mobile phone carried by the person (i.e. locally), foregoing the need for an external server.

Preferably, the personal sensing device is paired to a predetermined, specific user, such as by means of pairing via NFC, RFID or Bluetooth. Preferably, passive Bluetooth is used (i.e. "Passive Bluetooth Tags" - PBT). NFC, RFID or Bluetooth may also be used for localization purposes - or for identifying tools, equipment, installations, et cetera.

In an embodiment, the computer system may thus be controlled by a mobile telephone application on the mobile telephone of the person.

In an embodiment, the computer system is arranged on a server external to the personal sensing device, such as a server physically present in a control room or in general a computer system physically present in the control room. Multiple control rooms/multiple computer systems may even be coupled, e.g. on multiple sites. The computer systems may even be integrated in a (company-wide) incident management system/platform. Thus, when the mobile phone application or the computer system of the personal sensing device is no longer functioning properly, the instructions may be distributed by the external server, leading to an additional layer of safety. The computer system may also be distributed over multiple devices, such as the personal sensing device, the external server and/or the mobile telephone.

In an embodiment, the computer system is configured to distribute, preferably intelligently distribute, the one or more alarm/event instructions in response to the alarm to another personal sensing device in the vicinity of the personal sensing device. Thus, the persons wearing the another personal sensing device(s) - who are often relatively nearby - can immediately respond to the situation.

In an embodiment, the personal sensing device comprises one or more pre-programmed messaging buttons, each configured to send an alarm or a signal representative of the event or operational condition to the computer system and/or to another personal sensing device when the pre-programmed message button is pressed. Many persons will often be on the site, several of said persons, preferably all of said persons wearing a personal sensing device. Thus, it may now not only be possible to send messages from the personal sensing device to the computer system (and vice versa) passively, but it is also possible to send active messages from the personal sensing device to the computer system. Even more so, it may (also) be possible to actively send messages from one personal sensing device (worn by one person) to another personal sensing device (worn by another person).

Advantageously, such messages are sent by using pre-programmed messaging buttons. So, when a person on site wearing a personal sensing device feels the need to send a message regarding an event or operational condition, e.g. to his/her colleagues and/or to the communication system, e.g. when said person is in distress or notices an urgent situation, there is no need to type or record a message, but an instant, pre-programmed message can be sent. This allows for very fast communication.

Advantageously, the one or more pre-programmed messaging buttons may act as manual override for triggering an alarm, e.g. in case the gas sensor fails or is broken and the person wearing the personal sensing device thinks that the gas concentration has exceeded said pre-determined limit even though an alarm is not generated by the computer system.

Advantageously, when a person on the site wearing a personal sensing device receives a message, e.g. from one of the persons colleagues and/or from the computer system, said person may respond directly by using one of the pre-programmed messaging buttons.

For example, upon receiving a message a person wearing a personal sensing device may use one of the pre-programmed buttons to confirm the safe receipt of the message, and/or to confirm that he/she will respond according to the instructions received. When a person does not respond, this may e.g. be an indication that the personal sensing device worn by the person is malfunctioning and/or that something is wrong with the person.

For example, when a (first) message has been sent to multiple receivers (e.g. more than five, such as more than ten), after a pre-defined number of persons (e.g. 2 or 3) have confirmed that they will respond according to the instructions of the (first) message, a second message may be sent to the other persons having received the (first) message that action is no longer needed. This ensures that not too many people respond and that operation on site is not interrupted too much when a message is sent by one person.

According to the invention, the personal sensing device comprises at least two pre-programmed messaging buttons allowing at least two, or more, pre-programmed messages to be send to the communication system and/or to another personal sensing device. This allows different messages with different levels of urgency to be sent. One level of urgency may correspond to "direct action needed", e.g. when a person is in distress. Another level of urgency may correspond to "for informative purposes only", e.g. when a person notices something seems to be off, but the person judges that direct action is not needed. A further level of urgency may correspond to "confirmation", e.g. to confirm that a message has been received safe and sound. A yet further level of urgency may correspond to "reaction", e.g. to inform that reaction is taken based on a received message. Hence, a differentiation may be made between "more urgent" messages and "less urgent" messages.

In embodiments the messages may thus be "active" messages, informing the computer system and/or another person (via his/her personal sensing device) of new information, and/or the messages may thus be "reactive" messages, responding to a received message.

Advantageously, by allowing persons on site to send "active" messages, more information about the conditions on site may be gathered by the computer system. Not only passive information is gathered, but also information gathered by persons on site, i.e. "human-intelligence" may be gathered by the computer system.

The personal sensing device is preferable part of a "sniffer system".

In the context of this document, the expression "site" may relate to any site where one or more persons are present and where hazardous gasses are known to exist. The site may for instance be a chemical processing plant, a waste processing plant, a mine, an agricultural site, et cetera.

In the context of this document, "computer system" may broadly relate to a local system on the personal sensing device, but may also relate to an external, centralized computer system operated independently of the personal sensing device, e.g. a cloud-based system. Preferably, in case of an external computer system, each personal sensing device has an individual connection with the external computer system, e.g. via GPRS or LTE-M, as well as other "loT"-based communication technologies to safeguard connectivity, i.e. preferably the personal sensing devices do not connect via each other (i.e. do not connect via "mesh") to the external server, because in case of an emergency, nearby personal sensing devices may malfunction, which could be dangerous in lone worker situations. Also, as stated before, preferably longer range communication techniques are employed, such as 2G (GPRS), 3G (UMTS), 4G (LTE) or 5G (NR), i.e. "cellular" technologies. The benefit of using such longer range, cellular communication techniques with respect to shorter range communication techniques, such as Wi-Fi and Wi-Fi "mesh" (as disclosed and employed by e.g. WO 2018/048517 A1), is that the signal strength/penetration with longer range communication techniques is better than with shorter range communication techniques, such as Wi-Fi (due to the use of lower transmission frequencies - typically 700 - 2100 Mhz, such as 800 - 1900 Mhz for cellular technologies versus 2400 Mhz or 5000 Mhz for Wi-Fi technologies). Alternatively, long range communication techniques, such as LoRa or communication via satellite (e.g. Iridium), could also be employed to communicate with the external alarm server. In embodiments, one or more of the pre-programmed messaging buttons may be formed by physical buttons on the personal sensing device, which may be pressed by a user of the personal sensing device. In embodiments, one or more of the pre-programmed messaging buttons may be formed by icons on a touchscreen of the personal sensing device.

In an embodiment, the operational condition comprises an indication indicates that the personal sensing device is working in a normal operational mode thereof. This allows e.g. a person wearing the personal sensing device to perform some checks regarding the functioning of the personal sensing device (to check if it working properly) before entering the site. As the person enters the operational condition may indicate that he/she enters the site wearing a functioning personal sensing device. This makes it possible to keep track of all persons on site wearing a personal sensing device. Of course, if the preliminary checks (performed before entering the site) indicate that the personal sensing device is not working properly, another personal sensing device should be worn and/or the site should not be entered.

In an embodiment, the operational condition comprises an indication that the person wearing the personal sensing device is responding to an alarm generated at a position away from the current position of said person. For example, a person on the site may receive a message or instructions that an alarm is generated in the neighbourhood of his/her position, e.g. within a distance of 100 meters. The received message and instructions may further indicate that help is desired. The person receiving the message or instructions may then use a pre-programmed messaging button to indicate that he/she is able to provide said help. This allows a fast response time. The alarm may e.g. be triggered by another person on site (a colleague) using a pre-defined messaging button on his/her personal sensing device or the alarm may be triggered by another device.

In an embodiment, the operational condition comprises an indication that the person wearing the personal sensing device is in distress and needs help. In other words, one of the pre-programmed buttons may e.g. be an "alarm button". Even though an alarm may be triggered by the computer system, it is desirable when an alarm can also be triggered by a person on site, such that said person can call for help when help is needed by said person. Many situations can be thought of in which a person needs immediate help. For example when a personal injury is suffered from or when the person discovers a dysfunctional system/element on site which needs urgent attention/repair. Advantageously, the personal sensing device allows to call for such help in an easy and fast way.

In an embodiment, the operational condition comprises an indication that the person wearing the personal sensing device is absent from the site. For example when the person wearing the personal sensing device leaves the site for a break or at the end of a working day, he/she may indicate in a fast and efficient manner that the site is left. It is then easy to keep track of all persons on site, without any additional infrastructure.

In an embodiment, the operational condition comprises an indication that communication between the personal sensing device and the communication system is lost. For example, an alarm (e.g. a sound signal, a vibratory signal, or a visual signal) may be generated when communication between the personal sensing device and the computer system is lost. The alarm may be generated at the personal sensing device with which communication is lost, and/or by the computer system, e.g. at the screen of an operator, the operator possibly being located off-site. The computer system may then intelligently send instructions to another personal sensing device or several other personal sensing devices (worn by other persons on the site) to inform the other (person wearing the) personal sensing devices of the lost signal and to further investigate the lost signal. This allows e.g. to quickly and efficiently check upon (or confirm) the health status of persons on site.

In an embodiment, the alarm instructions are sent to all further personal sensing devices within 100 meters (or within any other range, such as within 75 meters, within 50 meters or within 25 meters) of said personal sensing device, such that the alarm instructions can be received by the persons wearing said personal sensing device, whom can act directly if this is desired.

In an embodiment, said alarm instructions are sent to one, two, three, four, five or more (or any other number) personal sensing devices closest by said personal sensing device, such that the instructions can be received by the persons wearing said personal sensing device, whom can act directly if this is desired. However, the instructions may also be sent to any other number of personal sensing devices closest by said personal sensing device.

In an embodiment, said alarm instructions are sent to all further activated personal sensing devices on the site, allowing all persons on site (wearing a personal sensing device) to receive the alarm instructions sent by one person.

In an embodiment one of the pre-programmed buttons is a "confirmation button", triggering a pre-programmed messages indicating that the person wearing the personal sensing device has received and read an incoming message.

In an embodiment, the personal sensing device further comprises at least one of a screen, a camera, a speaker and a microphone, preferably a screen, a speaker and a microphone. This allows also other communication except for only pre-defined messages via messaging buttons.

In an embodiment, the personal sensing device further comprises at least another alarm, triggered by another trigger than the one alarm. For example, the another alarm may be triggered when the personal sensing device (and thus the person wearing it) enters a "hot zone" with a relative high concentration of the gas measured by the gas sensor.

In an embodiment, pressing the pre-programmed button once results in sending a first alarm or signal, representative of a first operational condition, and wherein pressing the pre-programmed button twice results in sending a second alarm or signal, representative of a second operational condition different from the first pre-programmed message. This advantageously allows to send several different pre-programmed messages while saving physical space on the personal detection device.

In an embodiment said gas sensor is a H₂S gas sensor. In alternative embodiments, the gas sensor may be a CO gas sensor, an O2 gas sensor or an NO_{X} sensor. In alternative embodiments, the personal sensing device may comprise more than one gas sensor. Preferably though, the gas sensor is embodied by a single, integrated sensor, configured for detecting a whole range of different gasses and/or other concerning or hazardous substances.

### BRIEF DESCRIPTION OF THE DRAWINGS

These and other features of the present invention will now be elucidated further with reference to the attached figures. In these Figures,
Figure 1 schematically illustrates a system of personal sensing devices according to the invention; and
Figure 2 schematically shows an embodiment of a personal sensing device according to the invention.

### DETAILED DESCRIPTION

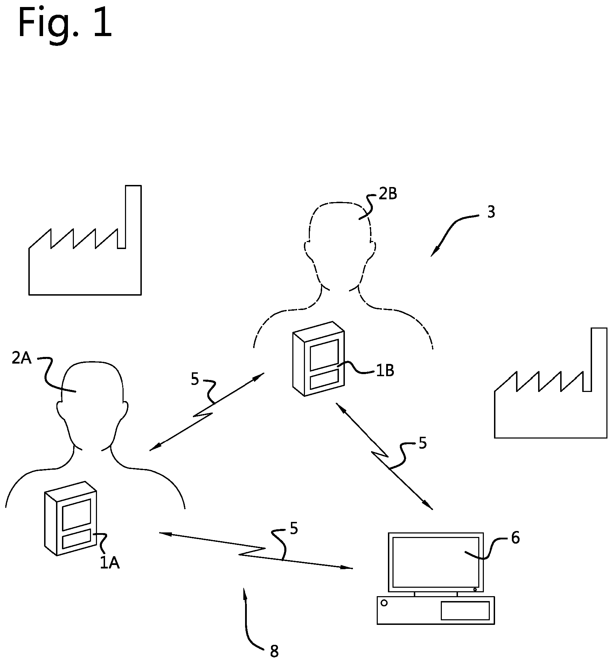

Figure 1 shows a system 8 for monitoring gas concentrations with personal sensing devices 1A, 1B, at a site 3, such as a chemical processing plant, a factory, et cetera. The personal sensing device 1A, 1B may be used outdoors, as well as indoors (e.g. in an enclosed area). Figure 1 shows two personal sensing devices 1A, 1B worn by two persons 2A, 2B. In principle, the personal sensing device 1A or 1B could also be arranged at a fixed position.

A communication system 5 is present for communicating an alarm/event or operational condition to a computer system 6 or, providing communication between the personal sensing devices 1A, 1B, such as "walkie talkie" functionality. When communication between the personal sensing devices 1A, 1B is provided, the computer system 6 may not be involved/may be left out in such communications. The communication system 5 may employ GPRS or LTE-M, as well as other "loT"-based communication technologies. Usually, the gas concentration is communicated to the computer system 6. Advantageously, persons 2A, 2B wearing a personal sensing device 1A, 1B may move around at the site 3, such that the measurement positions change in time and that different locations of the site 3 may be monitored. When the gas level at any one of the personal sensing devices 1A, 1B for instance reaches a predetermined threshold, an alarm may be triggered by the personal sensing device 1A, 1B or the computer system 6, and the computer system 6 then distributes, preferably intelligently distributes, one or more alarm/event instructions to, for instance to nearby personal sensing devices 1A, 1B to deal with the situation.

In general, the communication system 5 may therein employ two-way voice communication or text communication. The communication system 5 may also communicate internally within the personal sensing device 1A, 1B.

The personal sensing devices 1A, 1B are preferably used in a hands-free mode attached to a safety vest, gear harness, et cetera. An RFID/NFC/BT tag **may be used** in e.g. the gear harness to verify correct wear. The personal sensing devices 1A, 1B are easy to use, e.g. having a front side comprising a relatively large speaker 9 and a relatively large screen 10, and therefore do not require much training.

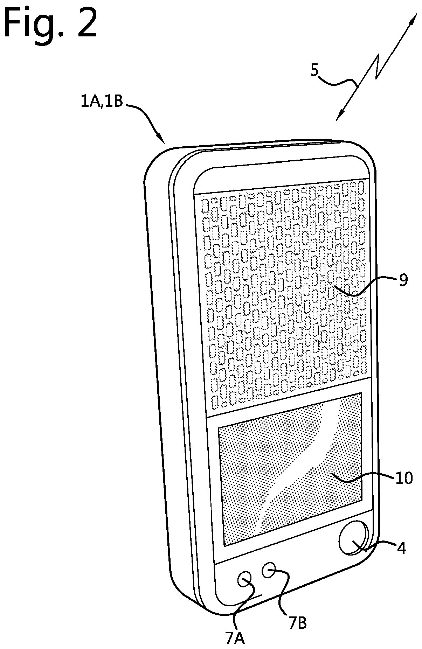

As shown in Figure 2, the personal sensing devices 1A, 1B comprise a gas sensor 4. The gas sensor 4 is configured for quantitative measurement of LEL (flammable/combustible), O₂, H₂S, CO and may contain indicative VOC measurement. Optional gasses that can be detected are SO₂, NO₂ and O₃ (by for instance changing one or more of the basic sensors and parameters in the software).

On "power-up" the personal sensing devices 1A, 1B can be paired to the person 2A, 2B which does turn every available personal sensing device 1A, 1B into a personal sensing device 1A, 1B that enables long term monitoring of the person 2A, 2B. This can be carried out by means of an RFID, NFC or a Bluetooth tag. As shown in Figure 2, an active display (pixels) may be used to enable showing menus and communication data. The alarm may be sent out or triggered when reaching (user configurable) exposure limits, such as:
∘ TWA - Time Weighed Average (8hrs)
∘ STEL - Short-Term Exposure Limit (15 minutes)
∘ C - Ceiling threshold levels

An alarm may also be triggered for an operational condition indicating that the battery is low, in case of communication (system 5) failure, blocked pump, man-down (no movement for a set time), fall detection. Preferably, the user must actively reset the alarm when triggered. The personal sensing device 1A, 1B preferably has a speaker 9 with voice and tone output at 90-95 dB at 10 cm, or pulsed output at 520 Hz, 1000 Hz, or 1600 Hz, such as by using a square wave. The tone and signal pattern are preferably user-configurable. The primary requirement for the alarm's warning signal is a sound pattern which makes the signal unambiguous. Preferably, an RGB LED is also flashing, in a user-configurable signal pattern. The personal sensing device 1A, 1B may also vibrate. Preferably, the communication system 5 is basically configured for sending time-stamped events to the computer system 6, where the communication/data may be stored. Alarm events are transmitted immediately. Testing events may also be stored and transmitted to the computer system 6. GPS data may be communicated when the position of the person 2A, 2B has changed by e.g. 10-50 meters (configurable) or when a change of Bluetooth beacon is detected. Gas concentrations may be logged, as well as movement info, position, wind direction, et cetera. Preferably, data is recorded at a 1 minute interval or less than a 1 minute interval, such as every 1 - 60 seconds, every 1 - 30 seconds, 1 - 10 or every 1 - 5 seconds. If the communication system 5 fails, data may be stored locally on the personal sensing device 1A, 1B. Of course, location/position determination systems similar to GPS, such as GNSS, may also be used for position determination purposes. Preferably, such a position determination system (i.e. regardless of the position determination system used) has a relatively high accuracy, e.g. of <= 2 m, such as <= 1 m, preferably <= 75 cm, such as 25 - 75 cm, preferably <= 50 cm, such as 25 - 50 cm. This facilitates position determination especially in lone worker situations. In particular, this facilitates position determination in case of altitude/height differences, such as in a building with multiple floors.

The computer system 6 is preferably configured to show a map with locations of all persons 2A, 2B and relevant information of all the locations, such as gas concentrations. Persons 2A, 2B may be members of groups based on function and location or persons 2A, 2B within a certain perimeter may be addressed based on real-time location information.

Regarding alarm events, on detection of gas STEL - Short-Term Exposure Limit overrun, a message may be sent via the communication system 5 to the computer system 6, such as an operational condition indicating the type of gas, concentration, location, movement, et cetera. On reaching C - Ceiling threshold levels threshold, a message may be sent to the computer system 6. On detection of gas TWA - Time Weighed Average overrun (integration of gas levels over time) a pre-warning may be sent to the computer system 6 when nearing the threshold.

On detection of no movement (time out), on detection of lying down (horizontal/programmable tilt angle) position, on detection of falling, on detection of unexpected (violent) movement, on detection of time out alarm, on pressing a button 7A, 7B, a pre-warning may be sent to the computer system 6, or if the personal sensing device 1A, 1B is not reset a message is sent to the computer system 6 or an alert is sent to other persons 2A, 2B of a group. The skilled person will understand that movement may be detected by a suitable movement sensor (not shown) included in the personal sensing device (1A, 1B).

On detection of change of location a new position may be sent to the computer system 6, or to other persons 2A, 2B within a group.

On detection of a "geo-fence" event (entering non-permitted areas), a message may be sent to the computer system 6, which may then send out instructions, preferably intelligent instructions, to handle the "geo-fence" event.

On detection of an empty battery, a message may also be sent to the computer system 6.

On detection of no viable connection via the communication system 5, such as no cellular connection, a message may be displayed on the screen 10. On reconnection, a message may be sent to the computer system 6.

On detection of sensor malfunctioning, below zero measurement, above maximum level measurement, maximum/minimum temperature, maximum humidity, a message may also be sent to the computer system 6.

On receiving an alarm, such as a gas event, a message may be sent to group members based on role of the receiving person 2A, 2B.

In case of a co-worker, the message may contain the name of the person sending the alarm, the type of alarm (gas type, measured level), the position of the alarm, the code or name of advised assembly point, or the evacuation route.

In case of an emergency response worker or first responder, the following information may be added to the message/intelligent alarm instructions: position to go to for assistance, advices route to go to person that needs assistance. This may also apply in case of a supervisor, medic, fireman, et cetera.

Preferably, all service actions are also logged by the computer system 6, such as physical check, calibration, replacement of sensors, batteries, et cetera.

The personal sensing device 1A, 1B may comprise a battery pack that is interchangeable and the personal sensing device 1A, 1B can be realized without rechargeable battery. Power sources may thus include an AA battery pack, a Li-lon battery with a capacity of 10 - 12 hrs or even up to 18 hrs. Wireless charging, such as according to the Qi standard, may also be employed.

Testing the personal sensing devices 1A, 1B may involve comparing with gas sensor 4 readings in the neighbourhood, such as from adjacent personal sensing devices 1A, 1B. This may require sending data from all the personal sensing devices 1A, 1B at set times, for instance every hour.

An extra gas sensor 4 may be included in the personal sensing device 1A, 1B as a redundant sensor to verify proper functioning, e.g. an additional H₂S sensor, for additional security.

A fixed embodiment of a personal sensing device 1A, 1B may comprise a continuous power supply and/or a particle sensor.

The personal sensing devices 1A, 1B preferably are designed to operate within a temperature range of -40 °C to +75 °C.

In Figure 1, two persons 2A, 2B are indicated on the site 3. Of course, many more persons may be present on the site 3. Advantageously, communication between these persons 2A, 2B is provided by the personal sensing device 1A, 1B worn by said persons 2A, 2B. As is visible in Figure 2, the personal sensing device 1A, 1B of the system 8 therefore comprises at least two, such as three, pre-programmed messaging buttons 7A, 7B. Each of the pre-programmed messaging buttons 7A, 7B is configured to send an alarm or signal representative of an operational condition to the computer system 6 or a specific pre-programmed message via the communication system 5 and/or to another personal sensing device 1B when it is pressed. Preferably, the pre-programmed messaging buttons 7A, 7B are large enough to be operable with a glove.

For example, an operational condition may indicate that the personal sensing device 1A, 1B is working in a normal operational mode thereof. For example, an operational condition may indicate that the person 2A, 2B wearing the personal sensing device 1A, 1B is responding to an alarm generated at a position away from the current position of said person 2A, 2B. For example, an operational condition may indicate that the person 2A, 2B wearing the personal sensing device 1A, 1B is in distress and needs help. For example, an operational condition may indicate that the person 2A, 2B wearing the personal sensing device 1A, 1B is absent from the site 3. For example, an operational condition may indicate that communication between the personal sensing device 1A, 1B via the communication system 5 is lost. For example, an operational condition may indicate that the person 2A, 2B wearing the personal sensing device 1A, 1B has received and read an incoming message.

For example, the alarm instructions are sent to all further personal sensing devices 1A, 1B within 100 meters of said personal sensing device 1A, 1B. For example, the alarm instructions are sent to five further personal sensing devices 1A, 1B closest by said personal sensing device 1A, 1B. For example, the alarm instructions are sent to all further activated personal sensing devices 1A, 1B on the site 3. For example, pressing the pre-programmed button 7A, 7B once results in sending a first alarm or signal, representative of a first operational condition, and wherein pressing the pre-programmed button 7A, 7B twice results in sending a second alarm or signal, representative of an operational condition different from the first operational condition.

The operational condition in general may also concern device health, device maintenance status, dosimeter status, hot spot identification, cold spot identification, process mining, et cetera.

The Applicant also foresees presentation of operational conditions or the one or more alarm instructions by means of augmented reality. Operational conditions or data may be presented via augmented reality e.g. on a wearable device, e.g. safety glasses or augmented reality glasses. Such operational conditions or the one or more alarm instructions may be made visible to a worker during his or her presence on the site, e.g. during his or her activities there. Auxiliary equipment, such as headphones, tablets, mobile phones, may also be used for such purposes.

Preferably, the personal sensing device 1A, 1B may be configured "over-the-air" (OTA), e.g. regarding alarm settings, alarm circles, alarm responders (assign, switch and exclude). The device may also be upgraded "OTA".

Device parameters may in general be set via the computer system 6 (e.g. the alarm server backend), preferably via mobile communication. A mobile communication application may be connected to the alarm server.

Preferably, the system has a monitor function, thereby displaying users/persons with active/absent status and actual location position.

As stated before, a team function may be included in the mobile application ("first responder phone users)", displaying team members 2A, 2B active/absent status and actual location position.

The computer system 6 preferably provides real-time insight in health status of the personal sensing device, covering bump tests, calibration and maintenance details for each personal sensing device 1A, 1B.

Advantageously, the computer system may comprise an online portal with administrator rights for user and group management and a unique settings profile per user group with "OTA" capabilities.

The communication system 5 preferably employs encrypted communication.

The computer system 6 may furthermore register time usage of each personal sensing device 1A, 1B per user 2A, 2B.

The computer system 6 preferably also provides real-time situational awareness in a control room. The computer system 6 may be physically arranged or present in the control room, or e.g. on a local server, on premise/on site, instead of via "cloud" (or in addition thereto).

It should be clear that the description above is intended to illustrate the operation of preferred embodiments of the invention, and not to reduce the scope of protection of the invention. Starting from the above description, many embodiments will be conceivable to the skilled person within the inventive concept and scope of protection of the present invention, which are defined by the appended claims.

### LIST OF REFERENCE NUMERALS

- 1.: Personal sensing device (1A, 1B)
- 2.: Person (2A, 2B)
- 3.: Site
- 4.: Gas sensor
- 5.: Communication system
- 6.: Computer system
- 7.: Message buttons (7A, 7B)
- 8.: System of personal sensing devices
- 9.: Speaker
- 10.: Screen

## Claims

1. Personal sensing device (1A, 1B) to be worn by a person (2A, 2B) on a site (3), comprising:
- a gas sensor (4) for measuring a gas concentration of a gas;
- a communication system (5) for communicating an alarm/event or operational condition, such as the gas concentration, to a computer system (6), wherein the computer system (6) is arranged for processing the communicated alarm/event and/or triggering an alarm/response in response to the communication of the operational condition, for instance when the computer system determines that the gas concentration exceeds a pre-determined limit, wherein the computer system (6) is configured for distribution of one or more alarm/event instructions in response to the alarm/event or operational condition, **characterized by**
- at least two pre-programmed messaging buttons allowing at least two different pre-programmed messages, with different levels of urgency, to be sent to the communication system and/or to another personal sensing device.

2. Personal sensing device (1A, 1B) according to claim 1, wherein the computer system (6) is comprised by the personal sensing device or a mobile phone carried by the person (2A, 2B) in communication with the personal sensing device.

3. Personal sensing device (1A, 1B) according to claim 2, wherein the computer system (6) is controlled by a mobile telephone application on the mobile telephone of the person (2A, 2B).

4. Personal sensing device (1A, 1B) according to claim 1, wherein the computer system (6) is arranged on a server external to the personal sensing device.

5. Personal sensing device (1A, 1B) according to any one of the preceding claims, wherein the computer system (6) is configured to distribute the one or more alarm instructions in response to the alarm to another personal sensing device (1B) in the vicinity of the personal sensing device (1A).

6. Personal sensing device (1A, 1B) according to any one of the preceding claims, comprising one or more pre-programmed messaging buttons (7A, 7B), each configured to send an alarm or a signal representative of the operational condition to the computer system (6) and/or to another personal sensing device (1A, 1B) when the pre-programmed message button (7A, 7B) is pressed.

7. Personal sensing device (1A, 1B) according to any one of the preceding claims,
wherein the operational condition comprises an indication that the personal sensing device (1A, 1B) is working in a normal operational mode thereof and/or
wherein the operational condition comprises an indication that the person (2A, 2B) wearing the personal sensing device (1A, 1B) is responding to an alarm generated at a position away from the current position of said person (2A, 2B) and/or
wherein the operational condition comprises an indication that the person (2A, 2B) wearing the personal sensing device (1A, 1B) is in distress and needs help and/or
wherein the operational condition comprises an indication that the person (2A, 2B) wearing the personal sensing device (1A, 1B) is absent from the site (3) and/or
wherein the operational condition comprises an indication that communication between the personal sensing device (1A, 1B) and the computer system (6) is lost and/or
wherein the operational condition indicates that the person (2A, 2B) wearing the personal sensing device (1A, 1B) has received and read an incoming message.

8. Personal sensing device (1A, 1B) according to any of the preceding claims, wherein the one or more alarm instructions are sent to all further personal sensing devices (1A, 1B) within 100 meters of the personal sensing device (1A, 1B), or wherein the one or more alarm instructions are communicated to one, two, three, four, five or more further personal sensing devices (1A, 1B) closest to said personal sensing device (1A, 1B).

9. Personal sensing device (1A, 1B) according to any of the preceding claims, wherein the one or more alarm instructions are sent to all further activated personal sensing devices (1A, 1B) on the site (3).

10. Personal sensing device (1A, 1B) according to any of the preceding claims, wherein the personal sensing device (1A, 1B) further comprises at least one of a screen, a camera, a speaker and a microphone.

11. Personal sensing device (1A, 1B) according to any of the preceding claims, comprising another alarm, triggered by another trigger than the one alarm.

12. Personal sensing device (1A, 1B) according any of the preceding claims, when dependent on claim 6, wherein pressing the pre-programmed button once results in sending a first alarm or signal, representative of a first operational condition, and wherein pressing the pre-programmed button twice results in sending a second alarm or signal, representative of a second operational condition different from the first operational condition.

13. Personal sensing device (1A, 1B) according to any of the preceding claims, wherein said gas sensor is a H₂S gas sensor.

14. Use of a personal sensing device (1A, 1B) according to any of the preceding claims.

15. System (8) of one or more personal sensing devices (1A, 1B) according to any one of the claims 1-13, a computer system (6) and a communication system (5) for communicating an alarm/event or an operational condition, such as a gas concentration, to the computer system (6), wherein the computer system is arranged for processing the communicated alarm/event and/or triggering an alarm/response in response to the communication of the operational condition of the personal sensing device, for instance when the computer system determines that the gas concentration exceeds a pre-determined limit, wherein the computer system (6) is configured for distribution of one or more alarm/event instructions in response to the alarm/event or operational condition.

## Patentansprüche

1. Persönliche Sensorvorrichtung (1A, 1B), die von einer Person (2A, 2B) an einem Ort (3) getragen wird und Folgendes umfasst:
- einen Gassensor (4) zum Messen einer Gaskonzentration eines Gases;
- ein Kommunikationssystem (5) zum Kommunizieren eines Alarms/Ereignisses oder eines Betriebszustands, wie etwa der Gaskonzentration, an ein Computersystem (6), wobei das Computersystem (6) zum Verarbeiten des kommunizierten Alarms/Ereignisses und/oder Auslösen eines Alarms/einer Antwort in Reaktion auf die Kommunikation des Betriebszustands ausgelegt ist, wenn beispielsweise das Computersystem bestimmt, dass die Gaskonzentration eine vorbestimmte Grenze überschreitet, wobei das Computersystem (6) zur Verteilung einer oder mehrerer Alarm-/Ereignisanweisungen in Reaktion auf den Alarm/das Ereignis oder den Betriebszustand konfiguriert ist, **gekennzeichnet durch**
- mindestens zwei vorprogrammierte Nachrichtenübermittlungstasten, die es ermöglichen, mindestens zwei verschiedene vorprogrammierte Nachrichten mit unterschiedlichen Dringlichkeitsstufen an das Kommunikationssystem und/oder eine andere persönliche Sensorvorrichtung zu senden.

2. Persönliche Sensorvorrichtung (1A, 1B) nach Anspruch 1, wobei das Computersystem (6) durch die persönliche Sensorvorrichtung oder ein von der Person (2A, 2B) getragenes Mobiltelefon in Kommunikation mit der persönlichen Sensorvorrichtung umfasst ist.

3. Persönliche Sensorvorrichtung (1A, 1B) nach Anspruch 2, wobei das Computersystem (6) durch eine Mobiltelefonanwendung auf dem Mobiltelefon der Person (2A, 2B) gesteuert wird.

4. Persönliche Sensorvorrichtung (1A, 1B) nach Anspruch 1, wobei das Computersystem (6) auf einem Server außerhalb der persönlichen Sensorvorrichtung angeordnet ist.

5. Persönliche Sensorvorrichtung (1A, 1B) nach einem der vorhergehenden Ansprüche, wobei das Computersystem (6) so konfiguriert ist, dass es die eine oder die mehreren Alarmanweisungen in Reaktion auf den Alarm an eine andere persönliche Sensorvorrichtung (1B) in der Nähe der persönlichen Sensorvorrichtung (1A) verteilt.

6. Persönliche Sensorvorrichtung (1A, 1B) nach einem der vorhergehenden Ansprüche, umfassend eine oder mehrere vorprogrammierte Nachrichtenübermittlungstasten (7A, 7B), die jeweils so konfiguriert sind, dass sie einen Alarm oder ein Signal, der/das den Betriebszustand darstellt, an das Computersystem (6) und/oder an eine andere persönliche Sensorvorrichtung (1A, 1B) senden, wenn die vorprogrammierte Nachrichtentaste (7A, 7B) gedrückt wird.

7. Persönliche Sensorvorrichtung (1A, 1B) nach einem der vorhergehenden Ansprüche,
wobei der Betriebszustand eine Anzeige umfasst, dass die persönliche Sensorvorrichtung (1A, 1B) in einem Normalbetriebsmodus davon arbeitet, und/oder
wobei der Betriebszustand eine Anzeige umfasst, dass die Person (2A, 2B), die die persönliche Sensorvorrichtung (1A, 1B) trägt, auf einen Alarm reagiert, der an einer Position entfernt von der aktuellen Position der Person (2A, 2B) erzeugt wird, und/oder
wobei der Betriebszustand eine Anzeige umfasst, dass die Person (2A, 2B), die die persönliche Sensorvorrichtung (1A, 1B) trägt, in Not ist und Hilfe benötigt, und/oder
wobei der Betriebszustand eine Anzeige umfasst, dass die Person (2A, 2B), die die persönliche Sensorvorrichtung (1A, 1B) trägt, nicht am Ort (3) anwesend ist, und/oder
wobei der Betriebszustand eine Anzeige umfasst, dass die Kommunikation zwischen der persönlichen Sensorvorrichtung (1A, 1B) und dem Computersystem (6) verloren gegangen ist, und/oder
wobei der Betriebszustand anzeigt, dass die Person (2A, 2B), die die persönliche Sensorvorrichtung (1A, 1B) trägt, eine eingehende Nachricht empfangen und gelesen hat.

8. Persönliche Sensorvorrichtung (1A, 1B) nach einem der vorhergehenden Ansprüche, wobei die eine oder die mehreren Alarmanweisungen an alle weiteren persönlichen Sensorvorrichtungen (1A, 1B) innerhalb von 100 Metern der persönlichen Sensorvorrichtung (1A, 1B) gesendet werden oder wobei die eine oder die mehreren Alarmanweisungen an eine, zwei, drei, vier, fünf oder mehr weitere persönliche Sensorvorrichtungen (1A, 1B) kommuniziert werden, die der persönlichen Sensorvorrichtung (1A, 1B) am nächsten sind.

9. Persönliche Sensorvorrichtung (1A, 1B) nach einem der vorhergehenden Ansprüche, wobei die eine oder die mehreren Alarmanweisungen an alle weiteren aktivierten persönlichen Sensorvorrichtungen (1A, 1B) am Ort (3) gesendet werden.

10. Persönliche Sensorvorrichtung (1A, 1B) nach einem der vorhergehenden Ansprüche, wobei die persönliche Sensorvorrichtung (1A, 1B) ferner mindestens eines von einem Bildschirm, einer Kamera, einem Lautsprecher und einem Mikrofon umfasst.

11. Persönliche Sensorvorrichtung (1A, 1B) nach einem der vorhergehenden Ansprüche, umfassend einen anderen Alarm, der durch einen anderen Auslöser als den einen Alarm ausgelöst wird.

12. Persönliche Sensorvorrichtung (1A, 1B) nach einem der vorhergehenden Ansprüche, wenn abhängig von Anspruch 6, wobei einmaliges Drücken der vorprogrammierten Taste zum Senden eines ersten Alarms oder Signals führt, der/das einen ersten Betriebszustand darstellt, und wobei zweimaliges Drücken der vorprogrammierten Taste zum Senden eines zweiten Alarms oder Signals führt, der/das einen zweiten Betriebszustand darstellt, der sich vom ersten Betriebszustand unterscheidet.

13. Persönliche Sensorvorrichtung (1A, 1B) nach einem der vorhergehenden Ansprüche, wobei der Gassensor ein H₂S-Gassensor ist.

14. Verwendung einer persönlichen Sensorvorrichtung (1A, 1B) nach einem der vorhergehenden Ansprüche.

15. System (8) aus einer oder mehreren persönlichen Sensorvorrichtungen (1A, 1B) nach einem der Ansprüche 1-13, einem Computersystem (6) und einem Kommunikationssystem (5) zum Kommunizieren eines Alarms/Ereignisses oder eines Betriebszustands, wie etwa einer Gaskonzentration, an das Computersystem (6), wobei das Computersystem zum Verarbeiten des kommunizierten Alarms/Ereignisses und/oder zum Auslösen eines Alarms/einer Antwort in Reaktion auf die Kommunikation des Betriebszustands der persönlichen Sensorvorrichtung ausgelegt ist, wenn beispielsweise das Computersystem bestimmt, dass die Gaskonzentration eine vorbestimmte Grenze überschreitet, wobei das Computersystem (6) zur Verteilung einer oder mehrerer Alarm-/Ereignisanweisungen in Reaktion auf den Alarm/das Ereignis oder den Betriebszustand konfiguriert ist.

## Revendications

1. Dispositif de détection personnel (1A, 1B) destiné à être porté par une personne (2A, 2B) sur un site (3), comprenant :
- un capteur de gaz (4) pour mesurer la concentration d'un gaz ;
- un système de communication (5) pour communiquer un(e) alarme/événement ou une condition opérationnelle, telle que la concentration de gaz, à un système informatique (6), dans lequel le système informatique (6) est agencé pour traiter l'alarme/événement communiqué(e) et/ou pour déclencher une alarme/réponse en réponse à la communication de la condition opérationnelle, par exemple lorsque le système informatique détermine que la concentration de gaz dépasse une limite prédéterminée, dans lequel le système informatique (6) est configuré pour la distribution d'une ou plusieurs instructions d'alarme/événement en réponse à l'alarme/événement ou à la condition opérationnelle, **caractérisé par**
- au moins deux boutons de messagerie préprogrammés permettant l'envoi d'au moins deux messages préprogrammés différents, avec différents niveaux d'urgence, au système de communication et/ou à un autre dispositif de détection personnel.

2. Dispositif de détection personnel (1A, 1B) selon la revendication 1, dans lequel le système informatique (6) est compris dans le dispositif de détection personnel ou dans un téléphone mobile porté par la personne (2A, 2B) en communication avec le dispositif de détection personnel.

3. Dispositif de détection personnel (1A, 1B) selon la revendication 2, dans lequel le système informatique (6) est commandé par une application de téléphone mobile sur le téléphone mobile de la personne (2A, 2B).

4. Dispositif de détection personnel (1A, 1B) selon la revendication 1, dans lequel le système informatique (6) est agencé sur un serveur externe au dispositif de détection personnel.

5. Dispositif de détection personnel (1A, 1B) selon l'une quelconque des revendications précédentes, dans lequel le système informatique (6) est configuré pour distribuer l'une ou les plusieurs instructions d'alarme, en réponse à l'alarme, à un autre dispositif de détection personnel (1B) à proximité du dispositif de détection personnel (1A).

6. Dispositif de détection personnel (1A, 1B) selon l'une quelconque des revendications précédentes, comprenant un ou plusieurs boutons de messagerie préprogrammés (7A, 7B), chacun configuré pour envoyer une alarme ou un signal représentatif/ive de la condition opérationnelle au système informatique (6) et/ou à un autre dispositif de détection personnel (1A, 1B) lorsque le bouton de messagerie préprogrammé (7A, 7B) est enfoncé.

7. Dispositif de détection personnel (1A, 1B) selon l'une quelconque des revendications précédentes,
dans lequel la condition opérationnelle comprend une indication que le dispositif de détection personnel (1A, 1B) fonctionne dans un mode opérationnel normal de celui-ci, et/ou
dans lequel la condition opérationnelle comprend une indication que la personne (2A, 2B) portant le dispositif de détection personnel (1A, 1B) répond à une alarme générée à une position éloignée de la position actuelle de ladite personne (2A, 2B), et/ou
dans lequel la condition opérationnelle comprend une indication que la personne (2A, 2B) portant le dispositif de détection personnel (1A, 1B) est en détresse et a besoin d'aide, et/ou
dans lequel la condition opérationnelle comprend une indication que la personne (2A, 2B) portant le dispositif de détection personnel (1A, 1B) est absente du site (3), et/ou
dans lequel la condition opérationnelle comprend une indication que la communication entre le dispositif de détection personnel (1A, 1B) et le système informatique (6) est perdue, et/ou
dans lequel la condition opérationnelle comprend une indication que la personne (2A, 2B) portant le dispositif de détection personnel (1A, 1B) a reçu et lu un message entrant.

8. Dispositif de détection personnel (1A, 1B) selon l'une quelconque des revendications précédentes, dans lequel l'une ou les plusieurs instructions d'alarme est/sont envoyée(s) à tous les autres dispositifs de détection personnels (1A, 1B) situés dans un rayon de 100 mètres du dispositif de détection personnel (1A, 1B), ou dans lequel l'une ou les plusieurs instructions d'alarme sont communiquées à un, deux, trois, quatre, cinq autres dispositifs de détection personnels ou plus (1A, 1B) qui sont les plus proches dudit dispositif de détection personnel (1A, 1B).

9. Dispositif de détection personnel (1A, 1B) selon l'une quelconque des revendications précédentes, dans lequel l'une ou les plusieurs instructions d'alarme est/sont envoyée(s) à tous les autres dispositifs de détection personnels activés (1A, 1B) sur le site (3).

10. Dispositif de détection personnel (1A, 1B) selon l'une quelconque des revendications précédentes, dans lequel le dispositif de détection personnel (1A, 1B) comprend en outre au moins un écran, une caméra, un haut-parleur et un microphone.

11. Dispositif de détection personnel (1A, 1B) selon l'une quelconque des revendications précédentes, comprenant une autre alarme, déclenchée par un autre déclencheur que l'une alarme.

12. Dispositif de détection personnel (1A, 1B) selon l'une quelconque des revendications précédentes, lorsqu'elle dépend de la revendication 6, dans lequel le fait d'appuyer une fois sur le bouton préprogrammé entraîne l'envoi d'une première alarme ou d'un premier signal, représentatif/ive d'une première condition opérationnelle, et dans lequel le fait d'appuyer deux fois sur le bouton préprogrammé entraîne l'envoi d'une seconde alarme ou d'un second signal, représentatif/ive d'une seconde condition opérationnelle différente de la première condition opérationnelle.

13. Dispositif de détection personnel (1A, 1B) selon l'une quelconque des revendications précédentes, dans lequel ledit capteur de gaz est un capteur de gaz H₂S.

14. Utilisation d'un dispositif de détection personnel (1A, 1B) selon l'une quelconque des revendications précédentes.

15. Système (8) d'un ou plusieurs dispositifs de détection personnels (1A, 1B) selon l'une quelconque des revendications 1 à 13, comprenant un système informatique (6) et un système de communication (5) pour communiquer un(e) alarme/événement ou une condition opérationnelle, telle qu'une concentration de gaz, au système informatique (6), dans lequel le système informatique est agencé pour traiter l'alarme/événement communiqué(e) et/ou pour déclencher une alarme/réponse en réponse à la communication de la condition opérationnelle du dispositif de détection personnel, par exemple lorsque le système informatique détermine que la concentration de gaz dépasse une limite prédéterminée, dans lequel le système informatique (6) est configuré pour la distribution d'une ou plusieurs instructions d'alarme/événement en réponse à l'alarme/événement ou à la condition opérationnelle.
